# FASCICULE DE BREVET EUROPEEN

(11) **EP 1 656 853 B1**
(45) Date de publication et mention de la délivrance du brevet: **27.02.2013**
(21) Numéro de dépôt: 05300925.4
(22) Date de dépôt: 15.11.2005
(51) Int. Cl.: A45D 40/24

(54) **Dispositif de conditionnement et de distribution d'au moins deux compositions différentes**
Vorrichtung zum Aufbewahren und Ausgeben von mindestens zwei unterschiedlichen Produkten
Device for storing and dispensing at least two different products

(30) Priorité: 15.11.2004 FR 0452620
(43) Date de publication de la demande: 17.05.2006
(73) Titulaire: L'Oréal, 75008 Paris (FR)
(72) Inventeur: Liechty, Anne, 75013, PARIS (FR); Hadasch, Anke, 75013, PARIS (FR)
(74) Mandataire: Tanty, François

(56) Documents cités:
- US-A- 5 111 972
- US-A- 6 116 466
- US-A1- 2004 221 864
- US-B1- 6 357 450

## Description

La présente invention concerne les dispositifs de conditionnement et de distribution d'au moins deux compositions différentes, notamment cosmétiques, stockées séparément et pouvant être mélangées ensemble lors de l'utilisation, en vue de leur application sur des matières kératiniques telles que la peau, les muqueuses ou les phanères.

Le brevet européen EP 0 758 615 décrit un dispositif permettant de disposer, dans un seul conditionnement, de plusieurs produits d'indices de protection solaire différents ou d'un produit de protection solaire et d'un après-soleil tel qu'une crème hydratante.

On connaît par ailleurs, notamment par les demandes de brevet européen EP 0 427 609, EP 1 040 773, le brevet US 5 971 210 ou le brevet US 4 893 729, d'autres dispositifs permettant de conditionner deux produits dans des réservoirs différents et de faire varier les proportions des produits dans le mélange distribué.

La société VERSADIAL Martkoberdorf propose un dispositif de distribution d'un mélange de deux produits solaires différents stockés séparément, ce dispositif comportant un organe de réglage permettant à un utilisateur de choisir la proportion relative de chaque produit dans le mélange distribué.

Le brevet américain US 5 013 244 décrit un kit comportant un premier dispositif stockant et distribuant un premier produit de protection solaire de faible indice de protection et un deuxième dispositif stockant et distribuant un deuxième produit de protection solaire d'indice élevé, coloré. Le kit comporte une échelle colorimétrique renseignant sur l'indice de protection solaire obtenu suivant la couleur du mélange des deux produits, que l'utilisateur peut effectuer dans sa main. L'échelle colorimétrique n'est pas destinée à être représentative de la couleur du mélange après application sur la peau.

La demande US 2004/0221864 divulgue un dispositif de distribution comportant deux solutions de coloration capillaire contenues dans des récipients différents et aptes à être mélangées au sein du dispositif.

Le brevet US 5 111 972 divulgue un dispositif de distribution comportant deux substances pâteuses, ces substances étant distribuées lors du déplacement d'un piston logé dans chaque récipient.

Le brevet US 6 116 466 décrit un dispositif de distribution de deux produits aptes à être mélangés comportant un organe de contrôle du flux d'un des produits, la proportion relative d'un produit au sein du mélange étant différente selon le mode de réalisation de l'organe de contrôle du flux.

Il existe un besoin pour bénéficier de nouveaux dispositifs de conditionnement et de distribution de produits cosmétiques.

L'invention vise notamment à répondre à ce besoin.

La présente invention a ainsi pour objet un procédé selon la revendication 1.

Par « effet optique visible », on désigne un effet optique qui peut être observé à l'oeil nu. La proportion relative précitée peut être capable, le cas échéant, de conditionner également une variation de couleur dans le mélange obtenu. La deuxième composition peut être capable, le cas échéant, de procurer également une variation de couleur dans le mélange obtenu.

Dans un exemple de mise en oeuvre de l'invention, la deuxième composition est capable de produire l'effet optique avec un degré variable, notamment avec différents degrés perceptibles par l'oeil humain, en fonction de sa proportion dans le mélange.

La deuxième composition peut par exemple comporter au moins un matériau, notamment des particules, ou une phase, contribuant partiellement ou totalement à l'obtention de l'effet optique visible autre que la couleur.

Selon cet aspect de l'invention, l'utilisateur peut agir à son gré au moins sur un effet optique autre que la couleur, produit par le mélange des deux compositions qu'il va appliquer sur la peau ou les muqueuses, notamment les lèvres, ou encore les phanères, par exemple les ongles, les cils ou les cheveux.

L'utilisateur pourra notamment adapter l'effet optique en fonction de sa tenue vestimentaire, du moment de la journée, de la météo ou de la saison, de l'occasion ou encore de son humeur ou de la région maquillée avec le produit.

Le dispositif peut éventuellement servir à plusieurs utilisateurs, par exemple au sein d'une famille, et chaque utilisateur pourra choisir le maquillage qui lui convient.

Les deux compositions qui sont rassemblées au sein du dispositif présentent avantageusement la même galénique ou des galéniques compatibles. Cela permet à un utilisateur d'associer une composition telle qu'un fond de teint ou une composition hydratante à une composition apportant par exemple un effet optique autre que la couleur, sans souci d'incompatibilité de galéniques, ce qui peut être le cas de plusieurs compositions conditionnées dans des dispositifs distincts, voire vendues par des sociétés différentes.

L'effet optique autre que la couleur peut être par exemple la couvrance, la variation de la couleur en fonction de l'angle d'observation, la diffraction de la lumière, l'inhomogénéité de l'aspect du mélange, la brillance ou encore la matité, cette liste n'étant pas limitative.

Dans un exemple de mise en oeuvre de l'invention, la première composition est capable de procurer au moins un effet optique visible, par exemple grâce à un matériau qu'elle comporte.

L'une au moins des première et deuxième compositions peut être dépourvue d'agent de coloration. L'une au moins des première et deuxième compositions peut comporter au moins un agent de coloration.

La première composition et la deuxième composition peuvent comporter chacune au moins un agent de coloration, par exemple le même agent de coloration, dans des concentrations différentes ou égales.

Le ou les agents de coloration contenus dans l'une ou dans les deux compositions peuvent être choisis dans le groupe constitué par les pigments minéraux, les pigments et laques organiques, les pigments nacrés, les pigments composites, les colorants liposolubles ou hydrosolubles, et leurs mélanges.

Lorsque les première et deuxième compositions comportent chacune plusieurs pigments, les proportions relatives de ces pigments entre eux au sein de chaque composition peuvent être sensiblement les mêmes, de manière à par exemple permettre de faire varier la couvrance sans faire varier sensiblement la couleur lorsque la proportion de la deuxième composition dans le mélange varie.

Dans le cas où l'effet optique est la couvrance ou la matité, la deuxième composition peut comporter au moins un pigment ou une charge, notamment une charge pouvant être choisie dans le groupe constitué par le talc, le mica, la silice, le kaolin, la séricite, les poudres de polyamide, de polyoléfines, notamment de polyéthylène, de polytétrafluoroéthylène, de polyméthacrylate de méthyle, de polyuréthane, les poudres d'amidon et les billes de résine de silicone.

Dans le cas où l'effet optique est la variation de la couleur en fonction de l'angle d'observation, la deuxième composition peut comporter au moins un agent de coloration goniochromatique, par exemple choisi dans le groupe constitué par les structures multicouche interférentielles et les agents de coloration à cristaux liquides.

Dans le cas où l'effet optique est la diffraction de la lumière, la deuxième composition peut comporter au moins un pigment diffractant.

Dans le cas où l'effet optique est l'inhomogénéité de l'aspect du mélange, la deuxième composition peut comporter des particules colorées ou réfléchissantes, visibles à l'oeil nu, par exemple des paillettes ou fibres.

Dans le cas où l'effet optique est la brillance, la deuxième composition peut par exemple comporter des particules réfléchissantes, des nacres ou des particules revêtues de métal, et/ou une phase huileuse brillante, selon par exemple la forme galénique ou la destination du mélange.

Les première et deuxième compositions peuvent être dépourvues de filtre UV. Par « filtre UV », on désigne au sens de la présente invention un matériau dont le spectre d'absorption se situe principalement dans le domaine des UVA et/ou des UVB.

La première composition peut par exemple être dépourvue d'agent de coloration et constituer une « base neutre », laquelle peut être blanche ou transparente, et la deuxième composition comporter par exemple au moins une nacre, ou au moins un agent de coloration et une nacre, ou encore au moins une charge et un agent de coloration, ou encore au moins un agent de coloration goniochromatique, ou un mélange de ceux-ci. En variante, la première composition peut comporter un agent de coloration et la deuxième composition comporter une nacre et/ou une charge, ou encore des particules réfléchissantes, ou un mélange de celles-ci.

Le dispositif peut être associé à une pluralité de compositions différentes, par exemple de couleurs différentes, pouvant être utilisées comme première composition. Le dispositif peut encore être associé à une pluralité de compositions différentes pouvant être utilisées comme deuxième composition.

Les première et deuxième compositions peuvent être des compositions cosmétiques, y compris de soin, notamment destinées à être appliquées sur les matières kératiniques, par exemple la peau, les muqueuses, notamment les lèvres, ou les phanères, notamment les ongles ou les fibres kératiniques. L'expression « composition cosmétique » englobe les compositions telles que définies dans la Directive 93/35/CEE du Conseil du 14 juin 1993. Les première et deuxième compositions selon l'invention comportent un milieu physiologiquement acceptable. Par « milieu physiologiquement acceptable », on désigne un milieu non toxique et susceptible d'être appliqué sur la peau, les lèvres ou les phanères d'êtres humains. Le milieu physiologiquement acceptable sera adapté à la nature du support sur lequel doit être appliqué le mélange, ainsi qu'à la forme sous laquelle les compositions sont destinées à être conditionnées, par exemple fluide à température ambiante et sous pression atmosphérique.

### Modes de mélange

Le mélange des première et deuxième compositions peut être réalisé à l'intérieur du dispositif, ou, en variante, les première et deuxième compositions peuvent être distribuées séparément, et être mélangées ou non à l'extérieur du dispositif

Lorsque le mélange est réalisé à l'intérieur du dispositif, ce dernier peut comporter une chambre de mélange. Cette dernière peut comporter un agitateur et/ou des canaux agencés pour faciliter le mélange des compositions.

Lorsque le mélange est réalisé à l'extérieur du dispositif, le mélange peut avoir lieu *in situ,* dans le creux de la main ou dans une coupelle, par exemple.

Le dispositif peut être agencé de manière à pouvoir distribuer simultanément ou successivement les première et deuxième compositions.

### Organe de réglage

L'organe de réglage du dispositif peut être ou non rotatif, l'expression « organe de réglage » ne devant pas être comprise limitativement. L'organe de réglage peut notamment englober un mécanisme comportant plusieurs éléments interagissant. L'organe de réglage peut, le cas échéant, être double, avec par exemple deux éléments de réglage pouvant être manoeuvrés indépendamment l'un de l'autre et agissant respectivement sur les quantités de chaque composition qui sont distribuées.

Notamment lorsque l'organe de réglage est unique, celui-ci peut comporter au moins deux positions correspondant à des proportions relatives différentes en première composition et en deuxième composition dans le mélange.

L'organe de réglage peut être configuré de manière à permettre, dans des positions extrêmes par exemple, à l'utilisateur de distribuer l'une des compositions seulement, ou encore l'une ou l'autre des compositions seulement.

L'organe de réglage peut être configuré pour permettre un réglage continu de la proportion de l'une des compositions dans le mélange, de manière à faire varier sa proportion par exemple entre 0 % et 100 %, au choix de l'utilisateur. En variante, l'organe de réglage peut être configuré pour permettre un réglage par incréments de la proportion de l'une des compositions dans le mélange, par exemple entre 0 % et 100 %, au choix de l'utilisateur, et comporter au moins deux positions de réglage discrètes, voire trois ou quatre positions ou davantage encore, chaque position correspondant à un mélange comportant une proportion prédéterminée en deuxième composition. L'organe de réglage peut être agencé pour émettre un clic ou présenter un point dur pour chaque position, afin de faciliter son positionnement par l'utilisateur dans une position donnée.

La course de l'organe de réglage entre deux positions successives peut représenter par exemple moins d'un quart de la course totale, entre deux positions extrêmes, de l'organe de réglage.

L'organe de réglage peut par exemple être agencé pour permettre d'obtenir après distribution un mélange contenant, pour une première position de l'organe de réglage, par exemple entre 0 % et 50 % en poids de la deuxième composition par rapport au poids total du mélange et pour une dernière position de l'organe de réglage, par exemple entre 50 % et 100 % en poids de la deuxième composition.

Par exemple, une position donnée de l'organe de réglage peut permettre d'obtenir un mélange contenant 90 % de première composition et 10 % de deuxième composition. Si l'utilisateur souhaite que l'effet optique soit différent, il pourra choisir une position de l'organe de réglage permettant d'obtenir un mélange contenant davantage de deuxième composition, par exemple 20 % ou 30 % en poids, ou plus encore.

Le dispositif peut comporter un bouton-poussoir unique pour distribuer le mélange ou un bouton-poussoir indépendant pour la distribution de chaque composition. Le ou les boutons-poussoirs peuvent agir sur des pompes ou valves, par exemple.

Le dispositif peut par exemple être agencé pour que la modification de la couvrance ou de l'homogénéité d'aspect ou d'autres effets optiques encore n'entraîne pas de modification sensible de la couleur du mélange, laquelle est par exemple sensiblement adaptée à la carnation de l'utilisateur.

Notamment lorsque c'est principalement la couvrance qui varie, l'organe de réglage peut comporter au moins deux positions de réglage successives et la variation de couleur ΔE (dans l'espace CIE Lab) du mélange entre ces deux positions successives de l'organe de réglage peut être inférieure ou égale à 0,8 environ, par exemple. La variation de couleur ΔE du mélange entre deux positions extrêmes de l'organe de réglage peut par exemple être inférieure ou égale à 2 environ, par exemple inférieure ou égale à 0,8.

Le dispositif peut être agencé pour délivrer au moins une information concernant la proportion relative des première et deuxième compositions dans le mélange en fonction du réglage choisi par l'utilisateur, et/ou au moins une information concernant au moins une propriété optique du mélange en fonction du réglage choisi par l'utilisateur.

### Modes de distribution et conditionnements

Le dispositif peut comporter, le cas échéant, un applicateur pour l'application du mélange. L'applicateur peut par exemple comporter une structure au moins partiellement élastiquement déformable, notamment une mousse, laquelle peut être floquée ou non.

En variante, le dispositif peut être dépourvu d'applicateur pour l'application du mélange, l'application pouvant alors s'effectuer directement sur la zone à maquiller ou après dépôt du mélange dans la paume de la main, sur un doigt, ou dans une coupelle par exemple.

Le dispositif peut être dépourvu de pompe, ou, au contraire, comporter au moins une pompe pour la distribution des compositions ou du mélange. Le dispositif peut par exemple comporter deux pompes indépendantes associées respectivement aux deux compositions. La ou les pompes peuvent être manuelles ou non, à reprise d'air ou sans reprise d'air.

Le mélange ou l'une au moins des compositions peut être distribué(e) le cas échéant sous la forme d'un spray.

Chacune des compositions est de préférence conditionnée dans un récipient du dispositif, à paroi souple ou non.

L'une au moins des première et deuxième compositions peut être contenue dans un récipient amovible ou non, du dispositif Les récipients peuvent être solidaires et constituer une recharge unitaire, l'ensemble des deux récipients pouvant être amovible. Les récipients contenant les compositions peuvent être pressurisés ou non.

Au moins une composition peut être contenue dans une poche souple disposée dans un récipient, ou être directement contenue dans un récipient sans l'intermédiaire d'une telle poche.

Les récipients contenant les compositions peuvent avoir les mêmes contenances ou non. Par exemple, le récipient contenant la première composition peut avoir une contenance supérieure ou égale au double, voire au triple ou plus de celle du récipient contenant la deuxième composition.

Au moins un récipient peut être au moins partiellement transparent ou non.

Au moins un récipient peut contenir, le cas échéant, une bille permettant d'homogénéiser son contenu.

Les récipients peuvent être juxtaposés ou être disposés autrement, notamment de façon concentrique, ou être superposés verticalement. De préférence, le dispositif est agencé pour tenir entièrement dans une main.

Il est également décrit un ensemble comportant :
- un dispositif de conditionnement et de distribution d'au moins une première composition et une deuxième composition différente de la première composition, les deux compositions étant aptes à être mélangées en vue de leur application sur des matières kératiniques, le dispositif étant agencé pour stocker séparément les première et deuxième compositions, l'une au moins des première et deuxième compositions contenant au moins un agent de coloration, le dispositif comportant un organe de réglage agencé pour permettre à un utilisateur de faire varier la proportion relative d'au moins une composition dans le mélange obtenu,
- au moins une information relative à un résultat visuel d'application du mélange sur des matières kératiniques, par exemple associée à au moins une position de l'organe de réglage, et/ou
- au moins une information relative à une couleur de peau, par exemple une couleur de peau pour laquelle au moins l'une des compositions est adaptée.

Par « résultat visuel d'application », on désigne l'aspect du mélange après application de celui-ci sur la surface qu'il est destiné à couvrir, par exemple la peau, les muqueuses ou les phanères. L'information relative au résultat visuel d'application peut par exemple renseigner sur la couleur du mélange telle qu'on peut l'observer après application sur ladite surface, dans des conditions normales d'application.

Selon cet aspect, ladite proportion relative peut conditionner la couleur du mélange et/ou un effet optique autre que la couleur.

Ainsi, seule la couleur du mélange peut par exemple varier en fonction de la position de l'organe de réglage.

L'ensemble peut par exemple comporter un atlas comportant au moins deux représentations représentant différentes apparences du produit selon la position de l'organe de réglage, par exemple différentes couleurs et/ou différents degrés de brillance, d'inhomogénéité d'aspect, d'intensité de la variation de la lumière en fonction de l'angle d'observation, d'intensité de la diffraction de la lumière ou de couvrance.

L'information, matérialisée par ces différentes représentations peut figurer directement sur le dispositif, par exemple sous la forme d'une échelle adaptée à coopérer avec un index de l'organe de réglage, ou sur l'emballage ou un support d'information, tel qu'une notice d'utilisation, associé au dispositif.

L'organe de réglage peut offrir au moins deux positions correspondant à des proportions relatives différentes en première et deuxième compositions dans le mélange, et à au moins une position de l'organe de réglage peut être associée au moins une information représentative d'un résultat visuel d'application. Une position de l'organe de réglage peut par exemple être associée à une marque colorée correspondant par exemple à la couleur de la peau de l'utilisateur ou à une couleur souhaitée.

Dans un exemple de réalisation, l'ensemble peut servir à délivrer un fond de teint en fonction du degré de bronzage de la peau de l'utilisateur. Par exemple, en début de saison estivale, le mélange pourra être plus clair qu'en fin de saison.

L'ensemble peut ainsi comporter, le cas échéant, des inscriptions telles « teint plus bronzé », « teint plus pâle », « teint naturel », associées par exemple à différentes positions de l'organe de réglage.

### Utilisations

Il est également décrit l'utilisation d'un dispositif ou d'un ensemble tel que défini plus haut pour le maquillage de la peau, l'une au moins des compositions étant un fond de teint.

Il est également décrit l'utilisation d'un dispositif ou d'un ensemble tel que défini plus haut pour le maquillage des lèvres, l'une au moins des compositions étant un rouge à lèvres liquide.

Il est également décrit l'utilisation d'un dispositif ou d'un ensemble tel que défini plus haut pour le maquillage des ongles, l'une au moins des compositions étant un vernis à ongles.

Il est également décrit l'utilisation d'un dispositif ou d'un ensemble tel que défini plus haut pour le maquillage des cils, l'une au moins des compositions étant un mascara.

Il est également décrit l'utilisation d'un dispositif tel que défini plus haut pour la coloration des fibres kératiniques, l'une au moins des compositions étant un produit de coloration.

### Procédés de maquillage

L'invention a encore pour objet un procédé de maquillage de matières kératiniques, par exemple de la peau, des lèvres ou des phanères, dans lequel on applique sur la peau, les lèvres ou les phanères, un mélange des deux compositions contenues dans le dispositif ou l'ensemble tel que défini plus haut.

L'une au moins des première et deuxième compositions peut par exemple être choisie en fonction de la couleur de la peau de l'utilisateur. Le cas échéant, au moins un paramètre de la couleur, notamment la clarté et/ou la teinte, de la peau de l'utilisateur peut être mesuré puis on délivre à l'utilisateur une recommandation concernant la première composition à utiliser, par exemple la couleur de celle-ci.

L'invention a encore pour objet, selon un autre de ses aspects, un procédé de maquillage de matières kératiniques, par exemple de la peau et/ou des phanères, à l'aide d'un dispositif ou d'un ensemble tel que défini plus haut, comportant les étapes suivantes :
- effectuer un premier réglage de l'organe de réglage,
- maquiller une première zone avec le mélange selon le premier réglage,
- effectuer un deuxième réglage de l'organe de réglage, différent du premier réglage,
- maquiller une deuxième zone avec le mélange selon le deuxième réglage.

L'invention a encore pour objet, selon un autre de ses aspects, un procédé de maquillage de matières kératiniques, par exemple de la peau, des lèvres ou des phanères, à l'aide d'un dispositif ou d'un ensemble tel que défini plus haut, comportant les étapes suivantes :
- choisir un résultat visuel,
- effectuer un réglage de l'organe de réglage visant à obtenir ce résultat,
- maquiller une zone de la peau, des lèvres ou des phanères avec le mélange selon ce réglage.

### Gammes de produits

Il est également décrit une gamme de premières et deuxièmes compositions colorées pouvant être utilisées dans un dispositif permettant de les mélanger dans une proportion choisie en fonction de la position d'un organe de réglage, par exemple dans un dispositif tel que défini plus haut, dans laquelle les différentes couleurs de la gamme permettent, seules ou en combinaison, d'obtenir sensiblement toutes les couleurs de peau d'une ethnie donnée, par exemple caucasienne, noire ou asiatique.

Par exemple, la gamme comporte des premières compositions ayant des couleurs différentes. Pour une première composition de la gamme, il existe dans la gamme une deuxième composition qui, pour une position donnée de l'organe de réglage, permet d'obtenir un mélange ayant sensiblement la couleur d'une autre première composition de la gamme. Ainsi, les plages de coloration obtenues avec les diverses associations de premières et deuxièmes compositions peuvent se recouvrir.

La gamme peut comporter par exemple plusieurs premières compositions ayant des tons d'une première dominante, par exemple jaune, avec plusieurs niveaux de clarté, et plusieurs deuxièmes compositions ayant des tons d'une deuxième dominante, par exemple rouge, avec plusieurs niveaux de clarté.

La gamme peut encore comporter, par exemple, plusieurs premières compositions ayant des tons clairs et plusieurs deuxièmes compositions ayant des tons foncés.

Il est également décrit une pluralité de premières et/ou de deuxièmes compositions, ayant des couleurs différentes, destinée à un dispositif permettant de les mélanger dans une proportion dépendant de la position d'un organe de réglage.

### Procédé de sélection

Il est également décrit un procédé de sélection de la première composition d'un dispositif ou d'un ensemble tel que défini plus haut, comportant les étapes suivantes :
- mesurer ou évaluer au moins un paramètre de la couleur de peau d'un utilisateur,
- sélectionner au moins une composition en fonction dudit paramètre.

### Couvrance

Dans le cas où au moins un effet optique procuré par la deuxième composition est la couvrance, la deuxième composition peut comporter au moins un pigment et/ou une charge.

La variation de couvrance procurée par la deuxième composition peut s'accompagner le cas échéant d'une variation de la matité, liée par exemple à la présence de la charge.

Par « charge », on désigne des particules de toute forme, insolubles dans le milieu de la composition, quelle que soit la température à laquelle la composition est fabriquée. Une charge peut servir notamment à modifier la rhéologie ou la texture de la composition. La nature et la quantité des particules pourra dépendre des propriétés optiques, mécaniques et des textures recherchées.

A titre d'exemple de charges, on peut citer, entre autres, le talc, le mica, la silice, le kaolin, la séricite, les poudres de polyamide, de polyoléfine, par exemple de polyéthylène, de polytétrafluoroéthylène, de polyméthacrylate de méthyle, de polyuréthane, les poudres d'amidon et les billes de résine de silicone.

En faisant varier la quantité d'au moins un pigment et/ou d'une charge dans le mélange, grâce à la variation de la proportion relative de la deuxième composition dans le mélange, on peut obtenir un mélange plus ou moins couvrant, c'est-à-dire présentant un pouvoir couvrant plus ou moins élevé. L'utilisateur peut donc choisir d'appliquer sur sa peau, ou ses lèvres ou encore ses phanères, un produit couvrant ou non, par exemple selon que la zone maquillée comporte ou non des défauts.

### Mesure du pouvoir couvrant

Pour mesurer le pouvoir couvrant d'une composition, on peut procéder de la manière suivante.

La composition est étalée avec une épaisseur de 30 µm sur une carte de contraste Erichsen, type 24/5, présentant un fond noir et un fond blanc, et l'on mesure la couleur à l'aide d'un colorimètre, par exemple de référence commerciale CR-300.

Des étalements similaires sont réalisés sur deux autres cartes de contraste et trois mesures sont effectuées sur chaque carte. La moyenne correspondant à ces neuf mesures est ensuite calculée.

Le pouvoir couvrant est inversement proportionnel à la variation de couleur (ΔE x 100) entre les mesures sur fond noir et sur fond blanc.

Lorsque la variation de couvrance est obtenue au moins en partie grâce à des pigments, les première et deuxième compositions peuvent comporter le ou les mêmes pigments, dans les mêmes proportions relatives au sein de chaque composition.

Cela peut permettre de limiter la variation de couleur éventuelle lors du mélange des deux compositions.

La variation du pouvoir couvrant 1/(ΔE x 100) entre deux positions de l'organe de réglage est par exemple comprise entre 1 et 50 %, mieux entre 1 et 30 %, et encore mieux entre 1 et 10 %.

### Variation de la couleur en fonction de l'angle d'observation

Lorsqu'au moins un effet optique procuré par la deuxième composition est la variation de la couleur en fonction de l'angle d'observation, la deuxième composition peut comporter au moins un agent de coloration goniochromatique.

Par « agent de coloration goniochromatique », on désigne au sens de la présente invention un agent de coloration permettant d'obtenir, lorsque la composition est étalée sur un support, un trajet de couleur dans le plan a*b* de l'espace colorimétrique CIE 1976 qui correspond à une variation Dh de l'angle de teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la deuxième composition ait été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.

Un agent de coloration goniochromatique au sens de la présente invention permet d'observer un changement de couleur, encore appelé « color flop », en fonction de l'angle d'observation.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques utilisables dans des compositions réalisées conformément à l'invention sont par exemple les structures suivantes : Fe₂O₃/SiO₂/Fe₂O₃/SiO₂/Fe₂O₃, un pigment ayant cette structure étant commercialisé sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celles commercialisées sous la dénomination HELICONE^{®} HC par la société WACKER.

Comme agent de coloration goniochromatique, on peut encore utiliser certaines nacres, des pigments à effets sur substrat synthétique, notamment substrat type alumine, silice, borosilicate, oxyde de fer, aluminium, ou des paillettes holographiques interférentielles issues d'un film de polytéréphthalate.

Le matériau peut en outre comporter des fibres goniochromatiques dispersées. De telles fibres pourront présenter une longueur inférieure à 80 µm par exemple.

La variation de la couleur en fonction de l'angle d'observation sera d'autant plus intense que la proportion relative en deuxième composition dans le mélange sera grande.

### Diffraction de la lumière

Lorsqu'au moins un effet optique procuré par la deuxième composition est la diffraction de la lumière, la deuxième composition peut comporter au moins un pigment diffractant.

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article *« Pigments Exhibiting Diffractive Effects* » d'Alberto Argoitia and Matt Witzman, 2002, Society of Vacuum coaters, 45^{th} Annual Technical Conference Proceedings 2002.

Le pigment diffractant peut être réalisé avec des motifs ayant différents profils, notamment triangulaires, symétriques ou non, en créneaux, de largeur constante ou non, sinuosoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : MgF₂, SiO₂, Al₂O₃, AlF₃, CeF₃, LaF₃, NdF₃, SmF₂, BaF₂, CaF₂, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs matériaux, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : MgF₂, SiO, SiO₂, Al₂O₃, TiO₂, WO, AIN, BN, B₄C, WC, TiC, TiN, N₄Si₃, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de SiO₂, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : MgF₂/Al/MgF₂, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du MgF₂ peut être comprise entre 80 et 95 % du poids total du pigment.

La quantité de pigment diffractant peut varier, en poids par rapport au poids total de la deuxième composition, par exemple de 0,1 à 5 %, voire de 0,5 à 5 %, ou encore de 0,5 % à 2,5 %, par exemple de l'ordre de 1 %.

La dimension du pigment diffractant peut être comprise par exemple entre 5 et 200 µm, mieux entre 5 et 100 µm, par exemple entre 5 et 30 µm. Par « dimension », on désigne la dimension donnée par la distribution granulométrique statistique à la moitié de la population, dite D50.

L'épaisseur des particules de pigment diffractant peut être inférieure ou égale à 3 µm, mieux 2 µm, par exemple de l'ordre de 1 µm.

Le pigment diffractant pourra être choisi de telle sorte que l'on puisse observer dans la deuxième composition, pour un éclairage incident à 45° et une variation de l'angle d'observation comprise entre 30° et -10°, une variation Dh de l'angle de teinte de la deuxième composition, dans le plan CIE 1976, d'au moins 50°, mieux d'au moins 70°, voire au moins 80° ou 90°, voire au moins 100°.

La mesure est effectuée alors que la composition est étalée à l'aide d'un étaleur automatique avec une épaisseur de 150 µm sur le fond noir d'une carte de contraste conventionnelle, notamment de marque ERICHSEN et de référence Typ 24/5.

On se sert d'un spectrogonioréflectomètre, avec éclairage incident 45° et un illuminant D65. L'appareil est en mode « observateur 10° », le spectre analysé étant 400-700 nm (avec un pas 5 nm). Le spectrogonioréflectomètre utilisé est celui de marque INSTRUMENT SYSTEMS et de référence GON 360 GONIOMETER.

Un angle d'observation négatif correspond à une observation dans le demi plan opposé à celui d'où provient la lumière, par rapport à la normale à la surface illuminée.

Pour une application sur les ongles notamment, la variation Dh peut être de préférence d'au moins 180°, de préférence encore au moins 270°, voire environ 360° ou plus par exemple.

Pour un gloss, la variation Dh de l'angle de teinte de la deuxième composition pourra être d'au moins 90°, entre les angles d'observation 30° et -10°.

L'effet diffractant sera d'autant plus visible et intense que la proportion relative de deuxième composition dans le mélange sera importante. Ainsi, l'utilisateur peut choisir en agissant sur l'organe de réglage du dispositif de diffracter plus ou moins la lumière.

### Variation de l'homogénéité d'aspect du mélange

Lorsqu'au moins un effet optique procuré par la deuxième composition est l'inhomogénéité d'aspect du mélange, la deuxième composition peut comprendre par exemple des particules réfléchissantes ou colorées, visibles à l'oeil nu, notamment des paillettes ou fibres.

### Variation de la brillance

Lorsqu'au moins un effet optique procuré par la deuxième composition est la brillance, la deuxième composition peut comprendre par exemple des particules réfléchissantes et/ou nacres ou encore une phase huileuse.

La brillance moyenne de la deuxième composition peut être supérieure à un certain seuil. Par « brillance moyenne », on désigne la brillance telle qu'elle peut être mesurée à l'aide d'un brillancemètre, de manière conventionnelle, par la méthode suivante.

### Mesure de la brillance moyenne

Sur une carte de contraste de marque LENETA et de référence FORM 1A PENOPAC, on étale une couche de 30 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER et de référence microTRI-GLOSS.

Les valeurs de brillance moyenne du mélange peuvent être comprises entre 0,01 et 60, par exemple, mieux entre 0,5 et 40, encore mieux entre 1 et 20.

### Particules réfléchissantes

Par « particules réfléchissantes », on désigne au sens de la présente invention des particules dont la taille, la structure, notamment l'épaisseur de la ou des couches qui la constituent et leur nature physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'oeil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes peuvent être présentes dans la deuxième composition à une teneur allant de 0,5 % à 60 % par rapport au poids total de la deuxième composition, notamment de 1 % à 30 % en poids, en particulier de 2 % à 20 % en poids, voire de 3 % à 10 % en poids.

Ces particules peuvent présenter des formes variées, notamment être en forme de plaquettes ou globulaires, en particulier sphériques.

Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.

Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un matériau métallique.

Des particules de verre recouvertes d'une couche métallique sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent, encore appelées « nacres blanches ».

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelle que soit leur forme, peuvent également être choisies parmi les particules à substrat synthétique enrobé au moins partiellement d'au moins une couche d'au moins un matériau métallique, notamment un oxyde métallique, choisi par exemple parmi les oxydes de titane, notamment TiO₂, de fer notamment Fe₂O₃, d'étain, de chrome, le sulfate de baryum et les matériaux suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS^{®} par la société ENGELHARD.

Les particules réfléchissantes peuvent être ou non goniochromatiques et/ou interférentielles ou non.

La deuxième composition selon l'invention peut comprendre au moins une nacre.

### Nacres

Par « nacre », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

### Huiles

Notamment lorsque les compositions sont destinées à être appliquées sur les lèvres, la deuxième composition peut comporter une phase huileuse conférant de la brillance, en particulier une phase huileuse présentant un indice de réfraction compris entre 1,36 et 1,56, mieux entre 1,36 et 1,50, encore mieux 1,37 à 1,49. L'indice de réfraction est mesuré à température ambiante (25°C), à l'aide d'un réfractomètre.

On peut choisir une phase huileuse telle que décrite dans la demande EP-A-792 637, dont le contenu est incorporé par référence dans la présente demande.

La deuxième composition peut contenir par exemple au moins une huile d'origine minérale, végétale ou synthétique, carbonée, hydrocarbonée, fluorée et/ou siliconée.

Par « huile hydrocarbonée », on entend des huiles contenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou des groupements alcool, éther, ester et/ou acide carboxylique.

Comme huiles utilisables, on peut ainsi citer, cette liste n'étant pas limitative, les huiles hydrocarbonées d'origine minérale ou synthétique telles les hydrocarbures linéaires ou ramifiés comme l'huile de paraffine ou ses dérivés, l'huile de vaseline, les polydécènes, le polyisobutène hydrogéné tel que le Parléam^{®} commercialisé par la société Nippon Oil Fats, le squalane d'origine synthétique ou végétale ; les huiles d'origine animale comme l'huile de vison, de tortue, le perhydrosqualène ; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, comme l'huile d'amande douce, de calophyllum, de palme, de pépins de raisin, de sésame, d'arara, de colza, de tournesol, de coton, d'abricot, de ricin, de luzerne, de courge, de cassis, de macadamia, de rosier muscat, de noisette, d'avocat, de jojoba, d'olive ou de germes de céréales (maïs, blé, orge, seigle) ; des esters d'acide gras et notamment d'acide lanolique, d'acide oléique, d'acide laurique, d'acide stéarique ; les esters de synthèse, comme par exemple l'huile de Purcellin (octanoate de cétostéaryle), l'isononanoate d'isononyle, le benzoate d'alcool en C₁₂ à C₁₅, le palmitate d'éthyl 2-hexyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyl-dodécyle, le succinate de 2-diéthyl-hexyle, le malate de diisostéaryle, le triisostéarate de glycérine ou de diglycérine ; les esters hydroxylés comme le lactate d'isostéaryle ; les esters du pentaérythritol ; les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alkoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ; les polysiloxanes modifiés par des acides gras, des alcools gras ou des polyoxyalkylènes comme les diméthicones copolyols ou les alkylméthicones copolyols ; les silicones fluorées liquides ; ou encore les triglycérides des acides caprylique/caprique comme ceux vendus par la société Stearineries Dubois ou ceux vendus sous les dénominations Miglyol 810, 812 et 818 par la société Dynamit Nobel ; et leurs mélanges.

Une bonne dispersion des pigments et/ou des charges, dans la deuxième composition, peut également permettre d'améliorer la brillance du mélange appliqué sur la zone à maquiller.

Dans le cas d'un vernis à ongle, la brillance peut être obtenue en introduisant dans la composition du vernis des composés, par exemple de type polyuréthane et latex.

La brillance pourra être d'autant plus intense que la proportion de la deuxième composition sera élevée dans le mélange.

L'utilisateur peut, en agissant sur l'organe de réglage, faire varier les proportions relatives des première et deuxième compositions en vue d'obtenir un mélange plus ou moins brillant ou plus ou moins mat ou satiné.

### Agents de coloration

L'une au moins des première et deuxième compositions peut comporter au moins un agent de coloration. La première composition et la deuxième composition peuvent comporter chacune au moins un agent de coloration, qui peut être le même agent de coloration dans des concentrations différentes ou égales.

La deuxième composition peut comporter un agent de coloration, afin par exemple d'obtenir lorsque mélangé à la première composition une variation de la couleur et/ou de la couvrance et/ou d'un autre effet optique visible.

L'agent de coloration peut par exemple être choisi parmi les pigments minéraux, les pigments ou laques organiques, les pigments nacrés, les pigments composites, les colorants liposolubles ou hydrosolubles.

Les pigments minéraux peuvent être blancs ou colorés, enrobés ou on. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Les pigments peuvent représenter de 0 à 40 %, de préférence de 1 à 35 %, et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % lorsque présents.

Les colorants liposolubles sont par exemple des extraits végétaux, le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont choisis par exemple parmi les extraits végétaux, notamment le jus de betterave et le bleu de méthylène.

Les colorants peuvent par exemple représenter de 0,1 à 20 % du poids de la première ou de la deuxième composition, voire de 0,1 à 6 %, lorsque présents.

L'agent de coloration peut comporter au moins une matière colorante organique, par exemple au moins un pigment organique et/ou au moins une laque organique.

La matière colorante organique peut être choisie par exemple parmi les matériaux particulaires insolubles dans le milieu physiologiquement acceptable de la composition.

La matière colorante organique peut comporter par exemple des pigments ou laques organiques qui peuvent être choisis parmi les matériaux ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association ».

L'agent de coloration peut comporter un pigment composite.

### Pigments composites

Le pigment composite peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

La première et/ou la deuxième composition peut comporter un ou plusieurs pigments composites tels que définis plus haut.

Le noyau inorganique du pigment composite peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

Le rapport de la plus grande dimension du noyau à sa plus petite dimension peut être compris entre 1 et 50.

Le noyau inorganique peut présenter une dimension comprise entre environ 1 nm et environ 100 nm, voire entre environ 5 nm et environ 75 nm, par exemple entre environ 10 nm et environ 50 nm.

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut être coloré, le cas échéant.

La matière colorante organique peut être telle que définie plus haut.

Le liant du pigment composite peut être de tout type dès lors qu'il permet à la matière colorante organique d'adhérer à la surface du noyau inorganique.

Le liant peut notamment être choisi parmi une liste non limitative comprenant les matériaux siliconés, les matériaux polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

### Autres ingrédients

Au moins l'une des première et deuxième compositions peut comporter d'autres ingrédients que ceux décrits plus haut, notamment au moins un solvant, une phase grasse, un polymère filmogène et/ou un actif dermatologique ou cosmétique, notamment en fonction de la forme galénique.

### Solvants

La première et/ou la deuxième composition peut comporter au moins un solvant aqueux ou organique.

Lorsque la première et/ou la deuxième composition comprend un ou plusieurs solvants organiques, ces solvants peuvent être présents en une teneur allant de 0,1 % à 99 %, par rapport au poids total de la composition concernée.

D'une manière générale, la quantité de solvant(s), notamment organique(s), dépendra de la nature du support sur lequel la composition est destinée à être appliquée.

La première et/ou la deuxième composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane ;

La première et/ou la deuxième composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La première et/ou la deuxième composition peut, en outre, contenir des éthers en C₂ et des aldéhydes en C₂-C₄ hydrophiles. L'eau ou le mélange d'eau et de solvants organiques hydrophiles peut être présent dans la première et/ou la deuxième composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### Phase grasse

La première et/ou la deuxième composition, par exemple lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes et leurs mélanges. La phase grasse peut, en outre, contenir des solvants organiques lipophiles.

La première et/ou la deuxième composition peut présenter par exemple une phase grasse continue, pouvant contenir moins de 5 % d'eau, notamment moins de 1 % d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

L'une au moins des compositions peut comporter un corps gras pâteux, une cire ou une gomme.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

### Polymère filmogène

La composition peut encore comporter, par exemple, un polymère filmogène, notamment dans le cas d'un mascara ou d'un vernis à ongles ou d'un fond de teint. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans une composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène, et éthyléniques, propyléniques ou butyléniques.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®,} NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEI KOGYO; ou encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER ; les sulfopolyesters vendus sous le nom de marque Eastman AQ par la société Eastman Chemical Products.

La première et/ou la deuxième composition selon l'invention peut comprendre également un agent auxiliaire de filmification favorisant la formation d'un film avec le polymère filmogène.

La première et/ou la deuxième composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...), autobronzants. Ces actifs peuvent être utilisés par exemple à des concentrations de 0 à 20 % et notamment de 0,001 à 15 % par rapport au poids total de la composition.

La première et/ou la deuxième composition peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, les colorants ou leurs mélanges.

La première et/ou la deuxième composition selon l'invention peut comprendre, selon le type d'application envisagée, les constituants classiquement utilisés dans les domaines considérés, qui sont présents en une quantité appropriée à la forme galénique souhaitée.

### Formes galéniques

La première et/ou la deuxième composition peut se présenter sous diverses formes, en fonction de sa destination. Chaque composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situés à l'interface huile/eau.

L'une des compositions, notamment la deuxième composition, peut se présenter sous la forme d'une poudre.

Chaque composition peut encore se présenter sous diverses autres formes, par exemple un gel.

Le mélange obtenu peut constituer une composition de maquillage, par exemple un rouge à lèvres, un gloss liquide, un fard à joues, un fond de teint fluide, un produit anti-cernes ou de contour des yeux, un eye-liner, un mascara, un vernis à ongles, une ombre à paupières fluide, un produit de maquillage du corps ou des cheveux ou encore un produit de coloration de la peau.

On va maintenant décrire en se référant au dessin des exemples de dispositifs convenant au conditionnement et à la distribution des première et deuxième compositions.
- la figure 1 est une vue en élévation, schématique, d'un exemple de dispositif conforme à l'invention,
- la figure 2 est une vue en élévation, schématique et partielle, d'une variante du dispositif de la figure 1,
- les figures 3 et 4 représentent en coupe axiale, schématique et partielle, un exemple de dispositif selon l'invention comportant un applicateur,
- la figure 5 représente de manière schématique un atlas pouvant être associé au dispositif selon l'invention,
- la figure 6 est une vue similaire à la figure 1 d'une variante de réalisation, et
- la figure 7 représente un exemple d'emballage.

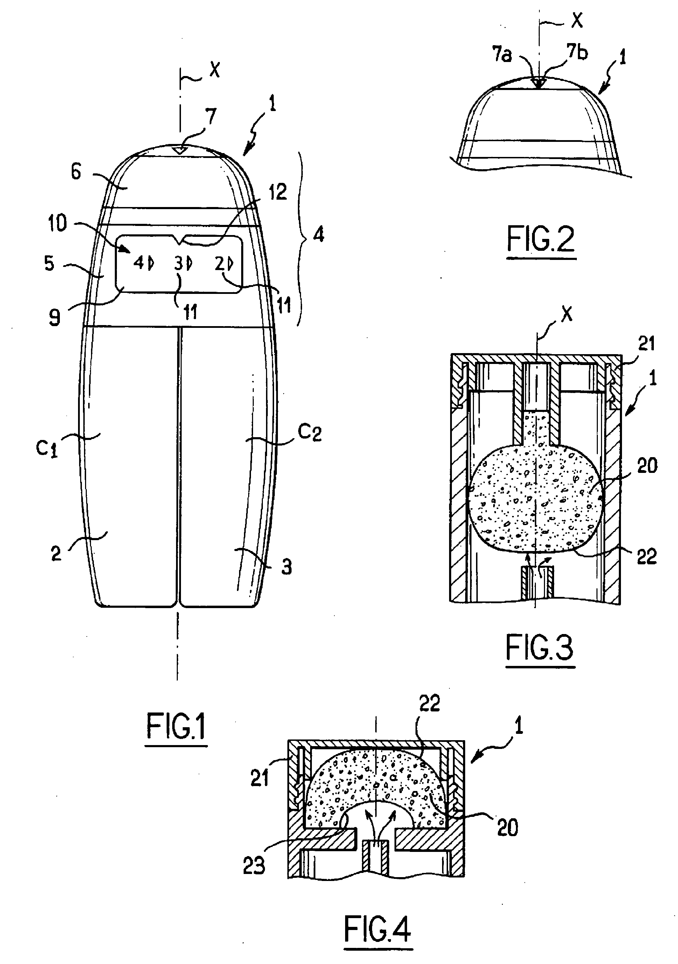

Le dispositif 1 représenté sur la figure 1 comporte deux récipients 2 et 3 contenant respectivement des première C₁ et deuxième C₂ compositions, destinées à être mélangées.

Le dispositif 1 comporte une tête de distribution 4 comportant dans l'exemple illustré une partie de base 5 fixe et un bouton-poussoir 6 mobile par rapport à la partie de base et agencé pour actionner une ou deux pompes, non visibles sur les figures. Le bouton-poussoir 6 est pourvu dans l'exemple considéré d'un orifice de distribution unique 7 pour distribuer le mélange des compositions C₁ et C₂.

L'orifice 7 peut être muni ou non d'un clapet afin de protéger les compositions de l'intrusion de tout élément extérieur.

La partie de base 5 peut comporter au moins une fenêtre 9, ou deux fenêtres diamétralement opposées dont une seule est visible sur la figure 1, donnant accès à un organe de réglage 10 permettant de régler les proportions des compositions C₁ et C₂ dans le mélange distribué.

L'organe de réglage 10 est dans l'exemple illustré rotatif autour de l'axe longitudinal X du dispositif 1 et comporte une série de positions 11, repérées par des chiffres par exemple, permettant à l'utilisateur, en positionnant l'une d'elle sous un repère 12 de la partie de base 5, de sélectionner un mélange à distribuer.

Dans l'exemple de la figure 1, le mélange des compositions C₁ et C₂ dans des proportions déterminées par le réglage de l'organe de réglage 10 est effectué à l'intérieur du dispositif 1, mais on ne sort pas du cadre de la présente invention lorsque le mélange est effectué après sortie du dispositif 1, celui-ci étant muni de deux orifices de distribution 7a et 7b pour distribuer respectivement les compositions C₁ et C₂ séparément, comme illustré sur la figure 2.

Comme on peut le voir sur les figures 3 et 4, le dispositif 1 peut être muni d'un applicateur 20 agencé pour recueillir le mélange et permettre à l'utilisateur de l'appliquer. Les applicateurs 20 représentés aux figures 3 et 4 sont constitués par des éléments élastiquement déformables, notamment des mousses poreuses.

On ne sort pas du cadre de la présente invention si l'applicateur comporte ou est constitué par tout autre élément, par exemple une brosse, un peigne, un pinceau, un embout floqué, un fritté, une lingette, un bloc de produit ou un applicateur retenant le produit par capillarité.

Dans l'exemple de la figure 3, l'applicateur est fixé à un couvercle 21 du dispositif et recueille le mélange distribué selon les flèches sur au moins une partie de sa surface extérieure 22.

Sur la figure 4, l'applicateur 20 est fixé sur le dispositif 1 de manière à recueillir le mélange distribué selon les flèches sur sa surface intérieure 23, le produit étant ensuite acheminé, par pression ou capillarité par exemple, jusqu'à la surface extérieure 22 de l'applicateur pour l'application.

On ne sort pas du cadre de la présente invention si l'applicateur est fixé de manière différente sur le dispositif 1, ou s'il a un autre usage.

En particulier, l'applicateur peut servir au mélange des deux compositions, ou au dosage de celles-ci. L'applicateur peut être à usage unique ou multiple.

L'un des récipients 2 et 3 ou les deux récipients peuvent être amovibles, de manière à pouvoir recharger le dispositif ou à le personnaliser en fonction par exemple de la peau ou des souhaits d'un utilisateur.

L'une au moins des première et deuxième compositions peut ainsi, par exemple, être sélectionnée dans une gamme de produits en fonction par exemple de la couleur de la peau de l'utilisateur.

On ne sort pas du cadre de la présente invention lorsque l'organe de réglage 10 est non rotatif.

D'autres dispositifs peuvent être utilisés, notamment ceux décrits dans la demande de brevet européen EP 1 040 773, le brevet US 5 568 883, le brevet US 5 971 210, le brevet US 4 893 729, ou encore le brevet US 5 143 261, qui sont incorporés à la présente par référence.

L'utilisateur peut bénéficier, le cas échéant, d'au moins une information représentative d'un résultat visuel du mélange, notamment pour une proportion relative prédéterminée des première et deuxième compositions C₁ et C₂ et/ou d'au moins une information relative à une couleur de peau à laquelle l'une au moins des compositions est adaptée.

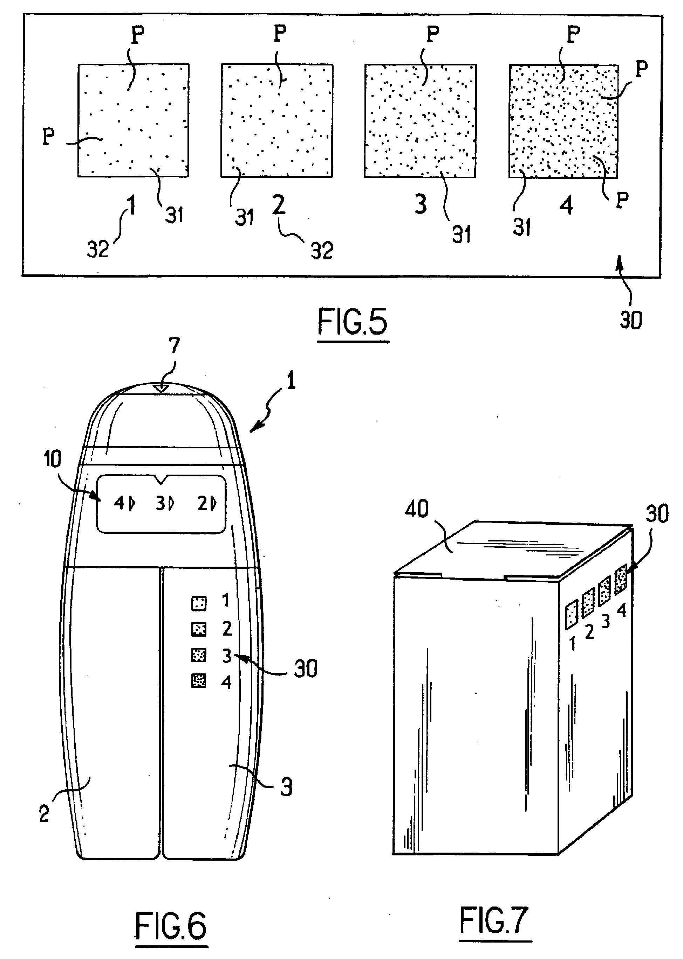

Le dispositif selon l'invention peut par exemple comporter un atlas 30 présentant plusieurs des caractéristiques du mélange par exemple sous forme de représentations 31, au nombre de quatre sur la figure 5.

Ces représentations 31 sont par exemple représentatives de résultats visuels que l'on peut obtenir en fonction de la proportion relative de la deuxième composition dans le mélange obtenu.

A chaque représentation 31 est associée une information 32 correspondant à un positionnement ou réglage donné de l'organe de réglage.

Dans l'exemple illustré, la deuxième composition C₂ comporte un matériau permettant de faire varier l'homogénéité d'aspect du mélange. Le matériau, dans cet exemple, comporte des particules P, par exemple des paillettes visibles à l'oeil nu ou non. Plus la proportion en deuxième composition est importante, plus la concentration en particules P est importante, plus l'aspect du mélange est pailleté et inhomogène. Si l'utilisateur choisit le réglage « 1 », il obtiendra un mélange dont l'aspect sera similaire à la représentation 31 associée au chiffre « 1 », à savoir faiblement pailleté. Si l'utilisateur choisit un réglage « 4 », il obtiendra un effet bien plus pailleté tel qu'illustré sur la représentation 31 associée au chiffre « 4 ».

L'atlas peut, dans une variante non illustrée, comporter au moins une représentation colorée représentative d'une couleur de peau à laquelle l'une au moins des compositions est adaptée, par exemple pour guider l'utilisateur dans son choix au sein d'une gamme de dispositifs ou de compositions selon l'invention suivant la couleur de sa peau.

Les représentations 31 ou autres marques ou représentations peuvent figurer sur une notice accompagnant le dispositif 1 ou encore être présentes directement sur le dispositif lui-même, par exemple sur l'un des récipients, comme illustré sur la figure 6.

Les représentations 31 peuvent encore être présentes sur un emballage 40 du dispositif 1, tel qu'illustré sur la figure 7, ou sur l'organe de réglage lui-même, à la place des inscriptions 11.

Bien sûr, l'atlas 30 peut être accompagné d'informations relatives à chaque position de l'organe de réglage ou à chaque résultat visuel, par exemple des explications sur l'effet optique obtenu selon la réglage choisi et/ou des conseils sur l'usage d'un mélange particulier, ou encore une information renseignant sur la proportion relative de chaque composition dans le mélange pour une position de l'organe de réglage donnée. La position « 1 » peut par exemple correspondre à un mélange contenant 90 % en poids de première composition et 10 % de deuxième composition, et la position « 4 » à un mélange contenant 30 % en poids de première composition et 70 % en poids de deuxième composition.

Le nombre de positions de l'organe de réglage peut être supérieur ou inférieur à quatre sans que l'on sorte du cadre de l'invention, le passage d'une position à l'autre pouvant le cas échéant s'effectuer de manière continue, permettant ainsi des positions intermédiaires correspondant à d'autres résultats. Les positions possibles de l'organe de réglage peuvent encore être autorisées par des crans.

Dans une variante non illustrée, la deuxième composition est apte à faire varier la couleur du mélange, en fonction de sa proportion dans le mélange.

Le dispositif peut alors comporter une échelle de couleurs coopérant avec un index de l'organe de réglage, chaque couleur de l'échelle correspondant à la couleur du mélange pour une proportion relative prédéterminée des compositions dans ce mélange.

### Exemples

Les exemples ci-après sont présentés à titre illustratif et non limitatif de l'invention.

Les compositions C₁ et C₂ peuvent être obtenues selon les procédés de préparation classiquement utilisés en cosmétique.

### Fonds de teint

| | **C₁** | **C₂** |
|---|---|---|
| **Exemple 1** | Base neutre ¹ | Composition couvrante ³ |
| **Exemple 2** | Base neutre ¹ | Composition nacrée ⁴ |
| **Exemple 3** | Base neutre ¹ | Composition goniochromatique ⁵ |
| **Exemple 4** | Base neutre ¹ | Composition colorée ⁶ |
| **Exemple 5** | Base colorée ² | Composition couvrante ³ |
| **Exemple 6** | Base colorée ² | Composition nacrée ⁴ |
| **Exemple 7** | Base colorée ² | Composition goniochromatique ⁵ |
| **Exemple 8** | Base colorée ² | Composition colorée ⁶ |
| **Exemple 9** | Base colorée ² | Composition colorée ⁷ couvrante |

Les quantités sont exprimées en masse dans tous les exemples.
^{1.} La base neutre peut par exemple présenter la formulation suivante :

| | |
|---|---|
| Butylène-1,3 glycol | 10 |
| Hectorite modifiée par chlorure de di-stéaryl di-méthyl ammonium (commercialisée sous le nom de Bentone 38 V par ELEMENTIS) | 1,6 |
| Conservateurs | 0,9 |
| Cyclopenta diméthylsiloxane | 15,6 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 est commercialisé par DOW CORNING) | 2 |
| Cétyl diméthicone copolyol (commercialisé sous la dénomination ABIL EM 90 par GOLDSCHMIDT) | 0,8 |
| Isostéarate polyglycérol | 0;6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB1 par NIHON JUNYAKU) | 2 |
| Poly diméthylsiloxane oxyéthyléné (commercialisé sous la dénomination KF-6017 de SHIN ETSU) | 5 |
| Eau | qsp 100 |

^{2.} La base colorée peut par exemple présenter la formulation suivante :

| | |
|---|---|
| Butylène-1,3 glycol | 10 |
| Hectorite modifiée par chlorure de di-stéaryl di-méthyl | 1,6 |
| ammonium (commercialisée sous le nom de Bentone 38 V par ELEMENTIS) | |
| Conservateurs | 0,9 |
| Cyclopenta diméthylsiloxane | 15,6 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 est commercialisé par DOW CORNING) | 2 |
| Cétyl diméthicone copolyol (commercialisé sous la dénomination ABIL EM 90 par GOLDSCHMIDT) | 0,8 |
| Isostéarate polyglycérol | 0,6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB 1 par NIHON JUNYAKU) | 2 |
| Poly diméthylsiloxane oxyéthyléné (commercialisé sous la dénomination KF-6017 de SHIN ETSU) | 5 |
| Oxyde de fer jaune enrobé de phosphate de perfluoroalkyle en dispersion dans décaméthyl cyclopentasiloxane/diméthicone copolyol (commercialisé sous la dénomination FA50DYF par KOBO) | 1,67 |
| Oxyde fer brun enrobé de phosphate de perfluoroalkyle en dispersion dans cyclométhicone/diméthyl polysiloxane copolyol (commercialisé sous la dénomination FA50DRF par KOBO) | 0,45 |
| Oxyde de fer noir enrobé de phosphate de perfluoroalkyle en dispersion dans décaméthyl clyclopentasiloxane/diméthicone copolyol (commercialisé sous la dénomination FA65DBF | 0,23 |
| par KOBO) | |
| Oxyde de titane traité alumine enrobé de phosphate de perfluoroalkyle dans décaméthyl cyclopentasiloxane/diméthicone copolyol (commercialisé sous la dénomination FA65DF par KOBO) | 5,52 |
| Eau | qsp 100 |

^{3.} La composition couvrante peut par exemple présenter la formulation suivante :

| | |
|---|---|
| Butylène-1,3 glycol | 10 |
| Hectorite modifiée par chlorure de di-stéaryl di-méthyl ammonium (commercialisée sous le nom de Bentone 38 V par ELEMENTIS) | 1,6 |
| Conservateurs | 0,9 |
| Cyclopenta diméthylsiloxane | 15,6 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 cst commercialisé par DOW CORNING) | 2 |
| Cétyl diméthicone copolyol (commercialisé sous la dénomination ABIL EM 90 par GOLDSCHMIDT) | 0,8 |
| Isostéarate polyglycérol | 0,6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB1 par NIHON JUNYAKU) | 2 |
| Poly diméthylsiloxane oxyéthyléné (commercialisé sous la dénomination KF-6017 de SHIN ETSU) | 5 |
| Oxyde de fer jaune enrobé de phosphate de perfluoroalkyle en dispersion dans décaméthyl cyclopentasiloxane/diméthicone copolyol (commercialisé sous la dénomination FA50DYF par KOBO) | 3,18 |
| Oxyde fer brun enrobé de phosphate de perfluoroalkyle en dispersion dans cyclométhicone/diméthyl polysiloxane copolyol (commercialisé sous la dénomination FA50DRF par KOBO) | 0,86 |
| Oxyde de fer noir enrobé de phosphate de perfluoroalkyle en dispersion dans décaméthyl clyclopentasiloxane/diméthicone copolyol (commercialisé sous la dénomination FA65DBF par KOBO) | 0,44 |
| Oxyde de titane traité alumine enrobé de phosphate de perfluoroalkyle dans décaméthyl cyclopentasiloxane/diméthicone copolyol (commercialisé sous la dénomination FA65DF par KOBO) | 10,52 |
| Eau | qsp 100 |

^{4.} La composition nacrée peut par exemple présenter la formulation suivante :

| | |
|---|---|
| Butylène-1,3 glycol | 10 |
| Hectorite modifiée par chlorure de di-stéaryl di-méthyl ammonium (commercialisée sous le nom de Bentone 38 V par ELEMENTIS) | 1,6 |
| Conservateurs | 0,9 |
| Cyclopenta diméthylsiloxane | 15,6 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 est commercialisé par DOW CORNING) | 2 |
| Cétyl diméthicone copolyol (commercialisé sous la dénomination ABIL EM 90 par GOLDSCHMIDT) | 0,8 |
| Isostéarate polyglycérol | 0,6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB1 par NIHON JUNYAKU) | 2 |
| Poly diméthylsiloxane oxyéthyléné (commercialisé sous la dénomination KF-6017 de SHIN ETSU) | 5 |
| Nacre | 8 |
| Eau | qsp 100 |

Une telle composition peut conférer de la brillance.
^{5.} La composition goniochromatique peut par exemple présenter la formulation suivante :

| | |
|---|---|
| Butylène-1,3 glycol | 10 |
| Hectorite modifiée par chlorure de di-stéaryl di-méthyl ammonium (commercialisée sous le nom de Bentone 38 V par ELEMENTIS) | 1,6 |
| Conservateurs | 0,9 |
| Cyclopenta diméthylsiloxane | 15,6 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 cst commercialisé par DOW CORNING) | 2 |
| Cétyl diméthicone copolyol (commercialisé sous la dénomination ABIL EM 90 par GOLDSCHMIDT) | 0,8 |
| Isostéarate polyglycérol | 0,6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB1 par NIHON JUNYAKU) | 2 |
| Poly diméthylsiloxane oxyéthyléné (commercialisé sous la dénomination KF-6017 de SHIN ETSU) | 5 |
| Pigment interférentiel | 8 |
| Eau | qsp 100 |

^{6.} La composition colorée peut par exemple présenter la formulation suivante :

| | |
|---|---|
| Butylène-1,3 glycol | 10 |
| Hectorite modifiée par chlorure de di-stéaryl di-méthyl ammonium (commercialisée sous le nom de Bentone 38 V par ELEMENTIS) | 1,6 |
| Conservateurs | 0,9 |
| Cyclopenta diméthylsiloxane | 15,6 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 cst commercialisé par DOW CORNING) | 2 |
| Cétyl diméthicone copolyol (commercialisé sous la dénomination ABIL EM 90 par GOLDSCHMIDT) | 0,8 |
| Isostéarate polyglycérol | 0,6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB1 par NIHON JUNYAKU) | 2 |
| Poly diméthylsiloxane oxyéthyléné (commercialisé sous la dénomination KF-6017 de SHIN ETSU) | 5 |
| Oxyde de fer jaune enrobé de phosphate de perfluoroalkyle en dispersion dans décaméthyl cyclopentasiloxane/diméthicone copolyol (commercialisé sous la dénomination FA50DYF par KOBO) | 2,7 |
| Oxyde fer brun enrobé de phosphate de perfluoroalkyle en dispersion dans cyclométhicone/diméthyl polysiloxane copolyol (commercialisé sous la dénomination FA50DRF par KOBO) | 1,1 |
| Oxyde de fer noir enrobé de phosphate de perfluoroalkyle en dispersion dans décaméthyl clyclopentasiloxane/diméthicone copolyol (commercialisé sous la dénomination FA65DBF par KOBO) | 1,4 |
| Oxyde de titane traité alumine enrobé de phosphate de perfluoroalkyle dans décaméthyl cyclopentasiloxane/diméthicone copolyol (commercialisé sous la dénomination FA65DF par KOBO) | 2,32 |
| Eau | qsp 100 |

^{7.} La composition colorée couvrante peut par exemple présenter la formulation suivante :

| | |
|---|---|
| Butylène-1,3 glycol | 10 |
| Hectorite modifiée par chlorure de di-stéaryl di-méthyl ammonium (commercialisée sous le nom de Bentone 38 V par ELEMENTIS) | 1,6 |
| Conservateurs | 0,9 |
| Cyclopenta diméthylsiloxane | 15,0 |
| Néopentanoate d'iso-stéaryle | 0,5 |
| Chlorure de sodium | 0,7 |
| Isododécane | 12,7 |
| Cyclohexadiméthylsiloxane | 7,7 |
| Poly diméthylsiloxane (DC 200 Fluid 5 cst commercialisé | 2 |
| par DOW CORNING) | |
| Cétyl diméthicone copolyol (commercialisé sous la dénomination ABIL EM 90 par GOLDSCHMIDT) | 0,8 |
| Isostéarate polyglycérol | 0,6 |
| Isoeicosane | 2 |
| Laurate d'hexyle | 0,6 |
| Microsphères creuses de polyméthacrylate de méthyle (commercialisées sous la dénomination COVABEAD LH85 par WACKHERR) | 2 |
| Poudre de polyméthylméthacrylate (commercialisée sous la dénomination JURYMER MB1 par NIHON JUNYAKU) | 2 |
| Poly diméthylsiloxane oxyéthyléné (commercialisé sous la dénomination KF-6017 de SHIN ETSU) | 5 |
| Oxyde de fer jaune enrobé de phosphate de perfluoroalkyle en dispersion dans décaméthyl cyclopentasiloxane/diméthicone copolyol (commercialisé sous la dénomination FA50DYF par KOBO) | 3 |
| Oxyde fer brun enrobé de phosphate de perfluoroalkyle en dispersion dans cyclométhicone/diméthyl polysiloxane copolyol (commercialisé sous la dénomination FA50DRF par KOBO) | 1,2 |
| Oxyde de fer noir enrobé de phosphate de perfluoroalkyle en dispersion dans décaméthyl clyclopentasiloxane/diméthicone copolyol (commercialisé sous la dénomination FA65DBF par KOBO) | 1,6 |
| Oxyde de titane traité alumine enrobé de phosphate de perfluoroalkyle dans décaméthyl cyclopentasiloxane/diméthicone copolyol (commercialisé sous la dénomination FA65DF par KOBO) | 2,32 |
| Eau | qsp 100 |

Les dispositifs des exemples 4 et 8 ou 9 sont par exemple fournis en kit, associés à au moins une information relative à une couleur de peau, par exemple une couleur de peau similaire à celle de l'utilisateur et/ou à celle souhaitée par l'utilisateur.

Dans l'exemple 1, selon la proportion de composition couvrante, on obtient un mélange plus ou moins couvrant. Dans l'exemple 2, selon la proportion de la composition nacrée, on obtient un mélange conférant plus ou moins de brillance à la peau. Dans l'exemple 3, selon la proportion de la composition goniochromatique, on obtient un mélange dans lequel l'effet goniochromatique est plus ou moins intense. Dans l'exemple 4, on obtient un mélange plus ou moins coloré. Les exemples 5 à 8 diffèrent des exemples 1 à 4 par la nature de la première composition. Dans l'exemple 9, on obtient en plus d'une variation de la couleur une variation de la couvrance plus importante que dans le cas de l'exemple 8.

Bien entendu, l'invention n'est pas limitée aux exemples qui viennent d'être décrits.

Le dispositif peut notamment être agencé pour conditionner et distribuer trois compositions différentes, ou plus.

Dans toute la description, y compris les revendications, l'expression « comportant un » doit être comprise comme étant synonyme de l'expression « comportant au moins un », sauf si le contraire est spécifié, et « compris entre » doit s'entendre bornes incluses.

## Revendications

1. Procédé de préparation d'une composition cosmétique en vue de son application sur des matières kératiniques par mélange d'au moins des première et deuxième compositions cosmétiques, différentes l'une de l'autre et stockées séparément dans un dispositif (1) de conditionnement et de distribution comportant un organe de réglage (10) permettant à l'utilisateur de faire varier la proportion relative d'au moins une composition dans le mélange obtenu, ladite proportion relative conditionnant au moins un effet optique visible autre que la couleur dans le mélange obtenu, ledit effet optique étant:
- la couvrance et la deuxième composition comportant une charge, ou
- la diffraction de la lumière et la deuxième composition comportant un pigment diffractant, ou
- l'inhomogénéité de l'aspect du mélange et la deuxième composition comportant des particules réfléchissantes, des paillettes ou fibres visibles à l'oeil nu ou une phase huileuse, ou
- la brillance et la deuxième composition comportant des particules réfléchissantes ou une phase huileuse,
l'organe de réglage comportant au moins deux positions correspondant à des proportions relatives différentes en première composition et en deuxième composition dans le mélange.

2. Procédé selon la revendication 1, **caractérisé par le fait que** la deuxième composition (2) comporte :
- des charges contribuant à la couvrance dans le mélange, ou
- des particules d'un pigment diffractant contribuant à la diffraction de la lumière dans le mélange, ou
- des particules réfléchissantes ou des paillettes ou fibres visibles à l'oeil nu contribuant à l'inhomogénéité de l'aspect du mélange, ou
- des particules réfléchissantes contribuant à la brillance dans le mélange.

3. Procédé selon la revendication 1, **caractérisé par le fait que** la deuxième composition comporte une phase huileuse contribuant à l'effet optique dans le mélange.

4. Procédé selon l'une quelconque des revendications précédentes, **caractérisé par le fait que** l'une au moins des première et deuxième compositions est dépourvue d'agent de coloration.

5. Procédé selon l'une quelconque des revendications 1 à 3, **caractérisé par le fait que** l'une au moins des première et deuxième compositions comporte un agent de coloration.

6. Procédé selon la revendication 5, **caractérisé par le fait que** la première composition et la deuxième composition comportent chacune au moins un agent de coloration.

7. Procédé selon la revendication précédente, **caractérisé par le fait que** les première et deuxième compositions comportent le même agent de coloration dans des concentrations différentes.

8. Procédé selon la revendication 6, **caractérisé par le fait que** les première et deuxième compositions comportent chacune plusieurs pigments et **par le fait que** les proportions relatives des pigments entre eux sont sensiblement égales au sein de chaque composition.

9. Procédé selon l'une quelconque des revendications 5 à 8, **caractérisé par le fait que** l'agent de coloration est choisi dans le groupe constitué par les pigments minéraux, les pigments et laques organiques, les pigments nacrés, les pigments composites, les colorants liposolubles ou hydrosolubles, et leurs mélanges.

10. Procédé selon l'une quelconque des revendications 1 à 9, **caractérisé par le fait que** la deuxième composition comporte au moins une charge.

11. Procédé selon la revendication précédente, **caractérisé par le fait que** la charge est choisie dans le groupe constitué par : le talc, le mica, la silice, le kaolin, la séricite, les poudres de polyamide, de polyoléfines, notamment de polyéthylène, de polytétrafluoroéthylène, de polyméthacrylate de méthyle, de polyuréthane, les poudres d'amidon et les billes de résine de silicone.

12. Procédé selon la revendication 1, **caractérisé par le fait que** la deuxième composition comporte un pigment diffractant et **par le fait que** l'effet optique est la diffraction de la lumière.

13. Procédé selon la revendication 1, **caractérisé par le fait que** la deuxième composition comporte des particules réfléchissantes et **par le fait que** l'effet optique est l'inhomogénéité de l'aspect du mélange ou la brillance.

14. Procédé selon la revendication 1, **caractérisé par le fait que** la deuxième composition comporte des paillettes ou fibres visibles à l'oeil nu et **par le fait que** l'effet optique est l'inhomogénéité de l'aspect du mélange

15. Procédé selon la revendication 1, **caractérisé par le fait que** la deuxième composition comporte une phase huileuse. et **par le fait que** l'effet optique est l'inhomogénéité de l'aspect du mélange ou la brillance.

16. Procédé selon l'une quelconque des revendications précédentes, **caractérisé par le fait que** les première et deuxième compositions sont dépourvues de filtre UV.

17. Procédé selon la revendication 2, **caractérisé par le fait que** la première composition est dépourvue d'agent de coloration et **par le fait que** la deuxième composition comporte une nacre.

18. Procédé selon la revendication 2, **caractérisé par le fait que** la première composition est dépourvue d'agent de coloration et **par le fait que** la deuxième composition comporte au moins un agent de coloration et une nacre.

19. Procédé selon la revendication 2, **caractérisé par le fait que** la première composition comporte un agent de coloration et **par le fait que** la deuxième composition comporte au moins une nacre.

20. Procédé selon la revendication 2, **caractérisé par le fait que** la première composition est dépourvue d'agent de coloration et **par le fait que** la deuxième composition comporte au moins une charge et un agent de coloration.

21. Procédé selon la revendication 2, **caractérisé par le fait que** la première composition comporte un agent de coloration et **par le fait que** la deuxième composition comporte une charge:

22. Procédé selon l'une quelconque des revendications précédentes, **caractérisé par le fait que** le mélange des première et deuxième compositions est réalisé à l'intérieur du dispositif.

23. Procédé selon l'une quelconque des revendications 1 à 21, **caractérisé par le fait que** les première et deuxième compositions sont distribuées séparément.

24. Procédé selon l'une quelconque des revendications précédentes, **caractérisé par le fait que** l'organe de réglage (10) est rotatif.

25. Procédé selon l'une quelconque des revendications précédentes, **caractérisé par le fait que** l'organe de réglage est configuré de manière à permettre à l'utilisateur de distribuer l'une des compositions seulement.

26. Procédé selon l'une quelconque des revendications précédentes, **caractérisé par le fait que** l'organe de réglage est configuré de manière à permettre à l'utilisateur de distribuer l'une ou l'autre des compositions seulement.

27. Procédé selon l'une quelconque des revendications précédentes, **caractérisé par le fait que** l'organe de réglage est configuré pour permettre un réglage continu de la proportion de l'une des compositions dans le mélange.

28. Procédé selon l'une quelconque des revendications précédentes, **caractérisé par le fait que** l'organe de réglage est configuré pour permettre un réglage par incréments de la proportion de l'une des compositions dans le mélange.

29. Procédé selon l'une quelconque des revendications précédentes, **caractérisé par le fait que** le dispositif est agencé de manière à pouvoir distribuer simultanément les première et deuxième compositions.

30. Procédé selon l'une quelconque des revendications précédentes, **caractérisé par le fait que** l'organe de réglage comporte au moins deux positions de réglage successives et **par le fait que** la variation de couleur ΔE du mélange entre ces deux positions successives de l'organe de réglage est inférieure ou égale à 0,8 environ.

31. Procédé selon la revendication précédente, **caractérisé par le fait que** la course entre deux positions successives représente moins d'un quart de la course totale de l'organe de réglage.

32. Procédé selon l'une quelconque des revendications précédentes, **caractérisé par le fait que** la variation de couleur ΔE du mélange entre deux positions extrêmes de l'organe de réglage est inférieure ou égale à 2 environ, notamment inférieure ou égale à 0,8 environ.

33. Procédé selon l'une quelconque des revendications précédentes, **caractérisé par le fait que** le dispositif est agencé pour délivrer au moins une information concernant la proportion relative des première et deuxième compositions dans le mélange en fonction du réglage choisi par l'utilisateur.

34. Procédé selon l'une quelconque des revendications précédentes, **caractérisé par le fait que** le dispositif est agencé pour donner au moins une information concernant au moins une propriété optique du mélange en fonction du réglage choisi par l'utilisateur, notamment une information relative à un résultat visuel d'application du mélange sur les matières kératiniques.

35. Procédé selon l'une quelconque des revendications précédentes, **caractérisé par le fait que** le dispositif comporte un applicateur pour l'application du mélange.

36. Procédé selon la revendication précédente, **caractérisé par le fait que** l'applicateur comporte une structure au moins partiellement élastiquement déformable, notamment une mousse.

37. Procédé selon l'une quelconque des revendications 1 à 34, **caractérisé par le fait que** le dispositif est dépourvu d'applicateur pour l'application du mélange.

38. Procédé selon l'une quelconque des revendications 1 à 37, **caractérisé par le fait que** le dispositif comporte au moins une pompe pour la distribution des compositions ou du mélange.

39. Procédé selon l'une quelconque des revendications précédentes, **caractérisé par le fait que** l'une au moins des première et deuxième compositions est contenue dans un récipient amovible du dispositif.

40. Procédé selon la revendication 39, **caractérisé par le fait que** le dispositif est associé à une pluralité de compositions différentes pouvant être utilisées comme première composition.

41. Procédé selon la revendication 39 ou 40, **caractérisé par le fait que** le dispositif est associé à une pluralité de compositions différentes pouvant être utilisées comme deuxième composition.

42. Procédé de maquillage des matières kératiniques, notamment de la peau, des lèvres ou des phanères, dans lequel on applique sur les matières kératiniques un mélange préparé selon le procédé tel que défini dans l'une quelconque des revendications 1 à 41.

43. Procédé selon la revendication 42, **caractérisé par le fait qu'**il comporte les étapes suivantes :
- effectuer un premier réglage de l'organe de réglage (10),
- maquiller une première zone avec le mélange selon le premier réglage,
- effectuer un deuxième réglage de l'organe de réglage (10) différent du premier réglage,
- maquiller une deuxième zone avec le mélange selon le deuxième réglage.

44. Procédé selon la revendication 42, **caractérisé par le fait qu'**il comporte les étapes suivantes :
- choisir un résultat visuel,
- effectuer un réglage de l'organe de réglage (10) visant à obtenir ce résultat,
- maquiller une zone de la peau, des lèvres ou des phanères avec le mélange selon le réglage effectué.

45. Procédé selon l'une quelconque des revendications 42 et 43, le mélange étant appliqué sur la peau, les lèvres ou les ongles.

## Claims

1. Process for preparing a cosmetic composition for its use on keratin materials by mixing at least first and second cosmetic compositions, which are different from each other and stored separately in a conditioning and dispensing device (1) comprising a regulating member (10) enabling the user to vary the relative proportion of at least one composition in the mixture obtained, the said relative proportion conditioning at least one visible optical effect other than the colour in the mixture obtained, the said optical effect being:
- the coverage, and the second composition comprising a filler, or
- the scattering of light, and the second composition comprising a scattering pigment, or
- the inhomogeneity of the appearance of the mixture, and the second composition comprising reflective particles, glitter flakes or fibres that are visible to the naked eye or an oily phase, or
- the gloss, and the second composition comprising reflective particles or an oily phase,
the regulating member comprising at least two positions corresponding to different relative proportions of first composition and of second composition in the mixture.

2. Process according to Claim 1, **characterized in that** the second composition (2) comprises:
- fillers that contribute toward the coverage in the mixture, or
- particles of a scattering pigment that contribute toward the scattering of light in the mixture, or
- reflective particles or glitter flakes or fibres that are visible to the naked eye, which contribute toward the inhomogeneity of the appearance of the mixture, or
- reflective particles that contribute toward the gloss in the mixture.

3. Process according to Claim 1, **characterized in that** the second composition comprises an oily phase that contributes towards the optical effect in the mixture.

4. Process according to any one of the preceding claims, **characterized in that** at least one of the first and second compositions is free of colouring agent.

5. Process according to any one of Claims 1 to 3, **characterized in that** at least one of the first and second compositions comprises a colouring agent.

6. Process according to Claim 5, **characterized in that** the first composition and the second composition each comprise at least one colouring agent.

7. Process according to the preceding claim, **characterized in that** the first and second compositions comprise the same colouring agent in different concentrations.

8. Process according to Claim 6, **characterized in that** the first and the second compositions each comprise several pigments and **in that** the relative proportions of the pigments among each other are substantially equal within each composition.

9. Process according to any on of Claims 5 to 8, **characterized in that** the colouring agent is chosen from the group consisting of mineral pigments, organic pigments and lakes, nacreous pigments, composite pigments an liposoluble or water-soluble dyes, and mixtures thereof.

10. Process according to any one of Claims 1 to 9, **characterized in that** the second composition comprises at least one filler.

11. Process according to the preceding claim, **characterized in that** the filler is chosen from the group consisting of: talc, mica, silica, kaolin, sericite, polyamide powder, polyolefin powder, especially polyethylene powder, polytetrafluoroethylene powder, polymethyl methacrylate powder, polyurethane powder, starch powders and silicone resin beads.

12. Process according to Claim 1, **characterized in that** the second composition comprises a diffractive pigment and **in that** the optical effect is diffraction of light.

13. Process according to Claim 1, **characterized in that** the second composition comprises reflective particles and **in that** the optical effect is the inhomogeneity of the appearance of the mixture or the gloss.

14. Process according to Claim 1, **characterized in that** the second composition comprises flakes or fibres that are visible to the naked eye and **in that** the optical effect is the inhomogeneity of the appearance of the mixture.

15. Process according to Claim 1, **characterized in that** the second composition comprises an oily phase and **in that** the optical effect is the inhomogeneity of the appearance of the mixture or the gloss.

16. Process according to any one of the preceding claims, **characterized in that** the first and second compositions are free of UV-screening agent.

17. Process according to Claim 2, **characterized in that** the first composition is free of colouring agent and **in that** the second composition comprises a nacre.

18. Process according to Claim 2, **characterized in that** the first composition is free of colouring agent an **in that** the second composition comprises at least one colouring agent and a nacre.

19. Process according to Claim 2, **characterized in that** the first composition comprises a colouring agent and **in that** the second composition comprises at least one nacre.

20. Process according to Claim 2, **characterized in that** the first composition is free of colouring agent and **in that** the second composition comprises at least one filler and a colouring agent.

21. Process according to Claim 2, **characterized in that** the first composition comprises a colouring agent and **in that** the second composition comprises a filler.

22. Process according to any one of the preceding claims, **characterized in that** the mixing of the first and second compositions is performed inside the device.

23. Process according to any one of Claims 1 to 21, **characterized in that** the first and second compositions are distributed separately.

24. Process according to any one of the preceding claims, **characterized in that** the regulating member (10) is rotary.

25. Process according to any one of the preceding claims, **characterized in that** the regulating member is configured so as to allow the user to distribute one of the compositions alone.

26. Process according to any one of the preceding claims, **characterized in that** the regulating member is configured so as to allow the user to distribute one or other of the compositions alone.

27. Process according to any one of the preceding claims, **characterized in that** the regulating member is configured to allow continuous setting of the proportion of one of the compositions in the mixture.

28. Process according to any one of the preceding claims, **characterized in that** the regulating member is configured to allow setting in increments of the proportion of one of the compositions in the mixture.

29. Process according to any one of the preceding claims, **characterized in that** the device is arranged so as to be able to simultaneously distribute the first and second compositions.

30. Process according to any one of the preceding claims, **characterized in that** the regulating member comprises at least two successive regulating positions and **in that** the variation in colour ΔE of the mixture between these two successive positions of the regulating member is less than or equal to about 0.8.

31. Process according to the preceding claim, **characterized in that** the course between two successive positions represents less than a quarter of the total course of the regulating member.

32. Process according to any one of the preceding claims, **characterized in that** the variation in colour ΔE of the mixture between two extreme positions of the regulating member is less than or equal to about 2 and especially less than or equal to about 0.8.

33. Process according to any one of the preceding claims, **characterized in that** the device is arranged to deliver at least one item of information concerning the relative proportion of the first and second compositions in the mixture as a function of the setting chosen by the user.

34. Process according to any one of the preceding claims, **characterized in that** the device is arranged to give at least one item of information retarding at least one optical property of the mixture as a function of the setting chosen by the user, especially information relating to a visual result of application of the mixture to the keratin materials.

35. Process according to any one of the preceding claims, **characterized in that** the device comprises an applicator for applying the mixture.

36. Process according to the preceding claim, **characterized in that** the applicator comprises an at least partially elastically deformable structure, especially a foam.

37. Process according to any one of Claims 1 to 34, **characterized in that** the device is not provided with an applicator for application of the mixture.

38. Process according to any one of Claims 1 to 37, **characterized in that** the device comprises at least one pump for distributing the compositions or the mixture.

39. Process according to any one of the preceding claims, **characterized in that** at least one of the first and second compositions is contained in a removable container of the device.

40. Process according to Claim 39, **characterized in that** the vice is combined with a plurality of different compositions that may be used as first composition.

41. Process according to Claim 39 or 40, **characterized in that** the device is combined with a plurality of different compositions that may be used as second composition.

42. Process for making up keratin materials, especially the skin, the lips or the integuments, in which a mixture prepared according to the process as defined in any one of Claims 1 to 41 is applied to the keratin materials.

43. Process according to Claim 42, **characterized in that** it comprises the following steps:
- setting the regulating member (10) to a first setting,
- making up a first area with the mixture according to the first setting,
- setting the regulating member (10) to a second setting different from the first setting,
- making up a second area with the mixture according to the second setting.

44. Process according to Claim 42, **characterized in that** it comprises the following steps:
choosing a visual result,
- setting the regulating member (10) to a setting aimed at obtaining this result,
- making up an area of the skin, the lips or the integuments with the mixture according to the selected setting.

45. Process according to any one of Claims 42 and 43, the mixture being applied to the skin, the lips or the nails.

## Patentansprüche

1. Verfahren zur Zubereitung einer kosmetischen Zusammensetzung im Hinblick auf deren Applikation auf keratinischen Materialien durch Mischung von zumindest von einer ersten und einer zweiten kosmetischen Zusammensetzung, von denen die eine von der anderen unterschiedlich ist und die getrennt in einer Vorrichtung (1) zur Konditionierung und zur Verteilung gelagert sind, die aufweist, ein Regelelement (10), das es dem Benutzer erlaubt, das relative verhältnis von zumindest einer Zusammensetzung in der erhaltenen Mischung zu verändern, wobei das besagte relative Verhältnis zumindest einen sichtbaren optischen Effekt ausmacht, der sich von der Farbe der erhaltenen Mischung unterscheidet, wobei der besagte optische Effekt ist:
- die Übertönung bzw. Überdeckung und die zweite Zusammensetzung weist einen Füllstoff auf, oder
- die Beugung bzw. Brechung des Lichtes und die zweite Zusammensetzung weist ein beugendes bzw. brechendes Pigment auf, oder
- die Ungleichmäßigkeit des Aussehens der Mischung und die zweite Zusammensetzung weist reflektierende Teilchen, Flitter bzw. Glitzerchen oder Fasern, die für das nackte Auge sichtbar sind, oder eine ölige Phase auf, oder
- der Glanz bzw. Hochglanz und die zweite Zusammensetzung weist reflektierende Teilchen oder eine ölige Phase auf,
wobei das Regelelement zumindest zwei Stellungen aufweist, die unterschiedlichen relativen Verhältnissen der ersten Zusammensetzung und der zweiten Zusammensetzung in der Mischung entsprechen.

2. Verfahren nach dem Anspruch 1, **dadurch gekennzeichnet, dass** die zweite Zusammensetzung (2) aufweist:
- Füllstoffe, die zu der Übertönung bzw. Überdeckung in der Mischung beitragen, oder
- Teilchen von einem beugenden bzw. brechenden Pigment, das zu der Beugung bzw. Brechung des Lichtes in der Mischung beiträgt, oder
- reflektierende Teilchen oder Flitter bzw. Glitzerchen oder Fasern, die für das nackte Auge sichtbar sind, die zu der Ungleichmäßigkeit des Aussehens der Mischung beitragen, oder
- reflektierende Teilchen, die zu dem Glanz bzw. dem Hochglanz in der Mischung beitragen.

3. Verfahren nach anspruch 1, **dadurch gekennzeichnet, dass** die zweite Zusammensetzung eine ölige Phase aufweist, die zu dem optischen Effekt in der Mischung beiträgt.

4. Verfahren nach irgendeinem der voranstehenden Ansprüche, **dadurch gekennzeichnet, dass** zumindest die eine der ersten und der zweiten Zusammensetzung ohne das Colorierungs- bzw. Färbungsmittel ist.

5. Verfahren nach irgendeinem der Ansprüche 1 bis 3, **dadurch gekennzeichnet, dass** zumindest die eine der ersten und zweiten Zusammensetzung ein Colorierungs- bzw. Färbungsmittel aufweist.

6. Verfahren nach anspruch 5, **dadurch gekennzeichnet, dass** die erste Zusammensetzung und die zweite Zusammensetzung jede zumindest ein Colorierungs- bzw. Färbungsmittel aufweisen.

7. Verfahren nach dem voranstehenden Anspruch, **dadurch gekennzeichnet, dass** die erste und die zweite Zusammensetzung das gleiche Färbungs- bzw. Colorierungsmittel in verschiedenen Konzentrationen aufweisen.

8. Verfahren nach anspruch 6, **dadurch gekennzeichnet, dass** die erste und die zweite Zusammensetzung jeweils mehrere Pigmente aufweisen, und dadurch, dass die relativen Verhältnisse der Pigmente unter einander innerhalb von jeder Zusammensetzung genau gleich sind.

9. Verfahren nach irgendeinem der Ansprüche 5 bis -8, **dadurch gekennzeichnet, dass** das Färbungs- bzw. Colorationsmittel aus der Gruppe ausgewählt ist, die aus mineralischen Pigmenten, den organischen Pigmenten und Lacken, den perlmuttartigen Pigmenten, den Pigmentzusammensetzungen, den fettlöslichen oder wasserlöslichen Farbstoffen und deren Mischungen,

10. Verfahren nach irgendeinem der Ansprüche 1 bis 9, **dadurch gekennzeichnet, dass** die zweite Zusammensetzung zumindest einen Füllstoff enthält.

11. Verfahren nach dem voranstehenden Anspruch, **dadurch gekennzeichnet, dass** das Füllmittel aus der Gruppe ausgewählt ist, die besteht aus: dem Talkum, dem Mika bzw. Glimmer, der Kieselerde, dem Kaolin, dem Sericid, den Pulvern des Polyamids, des Polyolefins, insbesondere des Polyethylens, des Polymethylmetacrylats, des Polyurethans, den Pulvem des Stärkemehls und den Kugeln bzw. Kügelchen des Harzes von Silikon.

12. Verfahren nach Anspruch 1, **dadurch gekennzeichnet, dass** die zweite Zusammensetzung brechende bzw. beugende Pigmente aufweist und dadurch, dass der optische Effekt die Brechung bzw. Beugung des Lichtes ist.

13. Verfahren nach Anspruch 1, **dadurch gekennzeichnet, dass** die zweite Zusammensetzung reflektierende Teilchen aufweist und dadurch, dass der optische Effekt die Inhomogenität bzw. Ungleichmäßigkeit des Aussehens der Mischung oder der Glanz bzw. Hochglanz ist.

14. Verfahren nach dem Anspruch 1, **dadurch gekennzeichnet, dass** die zweite Zusanmensetzung Kügelchen oder Fasern aufweist, die für das nackte Auge sichtbar sind, und dadurch, dass der optische Effekt die Ungleichmäßigkeit des Aussehens der Mischung ist.

15. Verfahren nach dem Anspruch 1, **dadurch gekennzeichnet, dass** die zweite Zusammensetzung eine ölige Phase aufweist, und dadurch, dass der optische Effekt die Ungleichmäßigkeit des Aussehens der Mischung oder der Glanz bzw. Hochglanz ist.

16. Verfahren nach irgendeinem der voranstehenden Ansprüche, **dadurch gekennzeichnet, dass** die erste und die zweite Zusammensetzung ohne einen UV-Filter sind.

17. Verfahren nach Anspruch 2, **dadurch gekennzeichnet, dass** die erste Zusammensetzung mit eine Colorierungs- bzw. Färbemittel versehen ist, und dadurch, dass die zweite Zusammensetzung ein Perlmutt aufweist.

18. Verfahren nach Anspruch 2, **dadurch gekennzeichnet, dass** die erste Zusammensetzung mit einem Mittel zur Coloration bzw. Färbung versehen ist, und dadurch, dass die zweite Zusammensetzung zumindest ein Mittel zur Coloration bzw. Färbung und ein Perlmutt aufweist.

19. Verfahren nach Anspruch 2, **dadurch gekennzeichnet, dass** die erste Zusammensetzung ein Mittel zur Coloration bzw. Färbung aufweist, und dadurch, dass die zweite Zusammensetzung zumindest ein Perlmutt aufweist.

20. Verfahren nach Anspruch 2, **dadurch gekennzeichnet, dass** die erste Zusammensetzung mit einem Mittel zur Coloration bzw. Färbung versehen ist, und dadurch, dass die zweite Zusammensetzung zumindest einen Füllstoff und ein Mittel zur Coloration bzw. Färbung aufweist.

21. Verfahren nach Anspruch 2, **dadurch gekennzeichnet, dass** die erste Zusammensetzung ein Mittel zur Coloration bzw. Färbung aufweist, und dadurch, dass die zweite Zusammensetzung einen Füllstoff aufweist.

22. Verfahren nach irgendeinem der voranstehenden Ansprüche, **dadurch gekennzeichnet, dass** die Mischung der ersten und zweiten Zusammensetzung im inneren der Vorrichtung verwirklicht wird.

23. Verfahren nach irgendeinem der Ansprüche 1 bis 21, **dadurch gekennzeichnet, dass** die erste und Zusammensetzung getrennt verteilt werden.

24. Verfahren nach irgendeinem der voranstehenden Ansprüche, gekennzeichnet, dass das Regelelement (10) drehbar ist.

25. Verfahren nach irgendeinem der voranstehenden Ansprüche, **dadurch gekennzeichnet, dass** das Regelelement in der Weise konstruiert ist, um es dem Benutzer zu ermöglichen, eine der Zusammensetzungen allein zu verteilen.

26. Verfahren nach irgendeinem der voranstehenden Ansprüche, **dadurch gekennzeichnet, dass** das Regelelement in der Weise konstruiert ist, um es dem Benutzer zu ermöglichen, die eine oder die andere der Zusammensetzungen allein zu verteilen.

27. Verfahren nach irgendeinem der voranstehenden Ansprüche, **dadurch gekennzeichnet, dass** das Regelelement konstruiert ist, um eine fortgesetzte Regelung des Verhältnisses von der einen der Zusammensetzungen in der Mischung zu ermöglichen.

28. Verfahren nach irgendeinem der voranstehenden Ansprüche, **dadurch gekennzeichnet, dass** das Regelelement konstruiert ist, um eine schrittweise Regelung des Verhältnisses der einen der Zusammensetzungen in der Mischung zu ermöglichen.

29. Verfahren nach irgendeinem der voranstehenden Ansprüche, **dadurch gekennzeichnet, dass** die Vorrichtung in der Weise ausgebildet ist, um es zu ermöglichen, die erste und die zweite Zusammensetzung gleichzeitig zu verteilen.

30. Verfahren nach irgendeinem der voranstehenden Ansprüche, **dadurch gekennzeichnet, dass** das Element zur Regelung zumindest zwei aufeinander folgende Stellungen zur Regelung aufweist, und dadurch, dass die Veränderung der Farbe ΔE von der Mischung zwischen diesen zwei aufeinander folgenden Stellungen des Regelelements kleiner oder gleich ungefähr 0,8 ist.

31. Verfahren nach dem voranstehenden Anspruch, **dadurch gekennzeichnet, dass** der Lauf bzw. die Strecke zwischen zwei aufeinander folgenden Stellungen weniger als ein Viertel des Gesamtlaufes bzw. der Gesamtstrecke des Regelelements darstellt.

32. Verfahren nach irgendeinem der voranstehenden Ansprüche, **dadurch gekennzeichnet, dass** die Veränderung der Farbe ΔE der Mischung zwischen zwei äußersten Stellungen des Regelelements weniger oder gleich ungefähr 2, insbesondere weniger oder gleich ungefähr 0,8 beträgt.

33. Verfahren nach irgendeinem der voranstehenden Ansprüche, **dadurch gekennzeichnet, dass** die Vorrichtung ausgebildet ist, um zumindest eine Information zu liefern, die das relative Verhältnis der ersten und der zweiten Zusammensetzung in der Mischung bezüglich der Funktion der von dem Benutzer gewählten Regelung betrifft.

34. Verfahren nach irgendeinem der voranstehenden Ansprüche, **dadurch gekennzeichnet, dass** die Vorrichtung ausgebildet ist, um zumindest eine Information zu geben, die zumindest eine optische Eigenschaft der Mischung bezüglich der Funktion der von dem Benutzer gewählten Regelung betrifft, insbesondere eine Information in Bezug auf ein sichtbares Ergebnis der Applikation der Mischung auf den keratinischen Materialien.

35. Verfahren nach irgendeinem der voranstehenden Ansprüche, **dadurch gekennzeichnet, dass** die Vorrichtung eine Applikationseinrichtung für die Applikation der Mischung aufweist.

36. Verfahren nach dem voranstehenden Anspruch, **dadurch gekennzeichnet, dass** die Applikationseinrichtung eine Struktur aufweist, die zumindest teilweise elastisch verformbar ist, insbesondere einen Schaum.

37. Verfahren nach irgendeinem der Ansprüche 1 is 34, **dadurch gekennzeichnet, dass** die Vorrichtung mit der Applikationseinrichtung zur Applikation der Mischung ausgebildet ist.

38. Verfahren nach irgendeinem der Ansprüche 1 bis 37, **dadurch gekennzeichnet, dass** die Vorrichtung zumindest eine Pumpe für die Verteilung der Zusammensetzungen oder der Mischung aufweist.

39. Verfahren nach irgendeinem der voranstehenden Ansprüche, **dadurch gekennzeichnet, dass** die zumindest eine der ersten und der zweiten Zusammensetzung in einem abnehmbaren Behälter der Vorrichtung enthalten ist.

40. Verfahren nach dem Anspruch 39, **dadurch gekennzeichnet, dass** die Vorrichtung mit mehreren verschiedenen Zusammensetzungen verbunden ist, die wie eine erste Zusammensetzung verwendet werden können.

41. Verfahren nach Anspruch 39 oder 40, **dadurch gekennzeichnet, dass** die Vorrichtung mit mehreren verschiedenen Zusammensetzungen verbunden ist, die als die zweite Zusammensetzung verwendet werden können.

42. Verfahren zum Schnünken von keratinischen Materialien, insbesondere der Haut, der Lippen oder der Hautanhanggebilde, bei welchem man auf den keratinischen Materialien eine Mischung appliziert, die gemäß dem Verfahren bereitet worden ist, wie dieses in irgendeinem der Ansprüche 1 bis 41 definiert ist.

43. Verfahren nach Anspruch 42, **dadurch gekennzeichnet, dass** es die folgenden Schritte aufweist:
- eine erste Regelung des Regelelements (10) wird bewirkt,
- eine erste Zone wird mit der Mischung gemäß der ersten Regelung geschminkt,
- eine zweite Regelung des Regelelements (10), die unterschiedlich von der ersten Regelung ist, wird bewirkt,
- eine zweite Zone wird mit der Mischung gemäß der zweiten Regelung geschminkt.

44. Verfahren nach dem Anspruch 42, **dadurch gekennzeichnet, dass** es die folgenden Schritte aufweist:
- ein sichtbares Ergebnis wird gewählt,
- eine Regelung des Regelelements (10), das die Erzielung dieses Ergebnisses anstrebt, wird bewirkt,
- eine Zone der Haut, der Lippen oder der Hautanhanggebilde wird mit der Mischung gemäß der bewirkten Regelung geschminkt.

45. Verfahren nach irgendeinem der Ansprüche, 42 und 43, wobei die Mischung auf die Haut, die Lippen oder die Nägel bzw. Fingernägel appliziert wird.
